# EUROPEAN PATENT SPECIFICATION

(11) **EP 3 828 545 B1**
(45) Date of publication and mention of the grant of the patent: **24.01.2024**
(21) Application number: 19868506.7
(22) Date of filing: 25.09.2019
(51) Int. Cl.: G01N 33/574, G01N 33/53, G01N 33/543

(54) **METHOD FOR ACQUIRING AUXILIARY INFORMATION**
VERFAHREN ZUR ERFASSUNG VON HILFSINFORMATIONEN
PROCÉDÉ D'ACQUISITION D'INFORMATIONS AUXILIAIRES

(30) Priority: 04.10.2018 JP 2018189367
(43) Date of publication of application: 02.06.2021
(73) Proprietor: Otsuka Pharmaceutical Co., Ltd., Tokyo 101-8535 (JP)
(72) Inventor: KANEKO, Tomonori, Tokyo 100-7015 (JP); KAYA, Takatoshi, Tokyo 100-7015 (JP); OHYAMA, Chikara, Hirosaki-shi, Aomori 036-8560 (JP); YONEYAMA, Tohru, Hirosaki-shi, Aomori 036-8560 (JP); TOBISAWA, Yuki, Hirosaki-shi, Aomori 036-8560 (JP); OGAWA, Osamu, Kyoto-shi, Kyoto 606-8501 (JP); INOUE, Takahiro, Kyoto-shi, Kyoto 606-8501 (JP)
(74) Representative: Gille Hrabal Partnerschaftsgesellschaft mbB Patentanwälte
(86) International application number: PCT/JP2019/037514
(87) International publication number: WO 2020/071199

(56) References cited:
- EP-A1- 3 358 352
- WO-A1-2015/097862
- WO-A1-2017/056844
- WO-A1-2018/101327
- WO-A1-2019/221279
- JP-A- 2009 142 284
- JP-A- 2013 531 997
- JP-A- 2016 519 767
- NIALL M. CORCORAN ET AL: "The ability of prostate-specific antigen (PSA) density to predict an upgrade in Gleason score between initial prostate biopsy and prostatectomy diminishes with increasing tumour grade due to reduced PSA secretion per unit tumour volume : ABILITY OF PSA DENSITY TO PREDICT AN UPGRADE IN GLEASON SCORE", BJU INTERNATIONAL, vol. 110, no. 1, 1 July 2012 (2012-07-01), pages 36-42, XP055547746, ISSN: 1464-4096, DOI: 10.1111/j.1464-410X.2011.10681.x
- GUINEVERE S. M. KAMMEIJER ET AL: "An In-Depth Glycosylation Assay for Urinary Prostate-Specific Antigen", ANALYTICAL CHEMISTRY, vol. 90, no. 7, 4 March 2018 (2018-03-04), pages 4414-4421, XP055675609, US ISSN: 0003-2700, DOI: 10.1021/acs.analchem.7b04281
- TOHRU YONEYAMA ET AL: "c-glycosylated Prostate-Specific Antigen Assay for prostate cancer detection", CANCER SCIENCE, 30 May 2019 (2019-05-30), XP055609286, JP ISSN: 1347-9032, DOI: 10.1111/cas.14082
- INOUE TAKAHIRO ET AL: "LacdiNAc-Glycosylated Prostate-specific Antigen Density is a Potential Biomarker of Prostate Cancer", CLINICAL GENITOURINARY CANCER, vol. 18, no. 1, 17 October 2019 (2019-10-17), pages e28-e36, XP055842242, US ISSN: 1558-7673, DOI: 10.1016/j.clgc.2019.10.011
- Shimuzu Tsukasa: "Examination of prostate-specific antigen density for differential diagnosis of prostatic hyperplasia and prostate cancer", Medical journal of Nagano Matsushiro General Hospital, vol. 22, 28 January 2010 (2010-01-28), pages 86-87, XP009526040, ISSN: 1342-291X
- MATSUO, EINOSHIN: "Detection of Prostate Carcinoma Using Prostate Specific Antigen, Its Density and the Density of the Transition Zone", Nagasaki medical journal, vol. 78, no. 3-4, 2003, pages 109-115, XP009526042, ISSN: 0369-3228
- UNO, HIROMI: "Clinical Significance of PSA-density in differential diagnosis between BPH and early stages prostate cancer", The Japanese Journal of Urolog, vol. 86, no. 12, 1995, pages 1776-1783, XP055653241,
- YAMADA, YOSHIAKI: "The Clinical Utility of PSA Density for Early Detection of Prostate Cancer in Japanese Patients with Inemediate Total PSA Levels", The journal of the Aichi Medical University Association, vol. 33, no. 3 / 4, 2005, pages 107-113, XP009526039, ISSN: 0301-0902

## Description

### Technical Field

The present invention relates to a method for acquiring auxiliary information on prostate cancer.

### Background Art

Prostate cancer mainly affects men over the age of 60, and is the most common cancer among men in the United States, with the highest number of morbidity and the second highest number of deaths. Particularly in Japan, the rate of increase thereof is remarkable, and according to the Cancer and Statistics White Paper (2012), the mortality rate of prostate cancer between 2020 and 2024 is predicted to be about 1.8 times that in 2000. Therefore, it is an urgent task in this field to accurately determine the presence or absence of prostate cancer and malignancy in the case of the incidence of prostate cancer.

In the diagnosis of prostate cancer or the like, first, screening using a prostate specific antigen (PSA) present in the patient's blood or the like is often performed. Examples of the screening using PSA include a method for estimating the incidence of prostate cancer by a method of quantifying the total PSA in a sample and comparing a value thereof with a threshold.

However, there is a problem that there are cases where false positives occur even though patients do not originally have cancer because the total PSA changes in blood concentration depending on the number of secreted prostate cells and the infiltration of cancer and tends to increase even in prostatic hyperplasia, which is a benign disease of prostate, so that the certainty of the total PSA screening in the treatment and diagnosis of prostate cancer is not high.

For example, if a total PSA value measured in a subject is 4 to 10 ng/mL, which is a so-called gray zone, it is determined that a detailed examination (so-called prostate needle biopsy), such as a histopathological diagnosis, to determine the degree of progression (stage), malignancy, and the like of cancer is necessary in many cases. In Japan, about 10 million PSA tests have been performed annually in recent years, of which less than 10% (less than 1 million people) exceed a reference value of 4 ng/mL, and it is determined that the detailed examination is necessary.

In reality, there are cases where the determination has changed due to the presence of subjects who do not undergo the detailed examination, re-examinations of PSA values, or the like. Thus, about 50% of the subjects who have been determined to require the detailed examination, that is, about 500,000 people actually undergo the needle biopsy. Among them, less than 90,000 people are affected by prostate cancer, and about 20% of subjects who have undergone the needle biopsy are found to have prostate cancer. In other words, it can be said that an unnecessary needle biopsy is performed for more than 80% (about 400,000 people) of the subjects who have undergone the needle biopsy.

Since the needle biopsy requires medical cost about 150,000 yen per person, the cost of the examination alone causes an annual economic loss of 60 billion yen, and further, costs human resources such as doctors, nurses, and laboratory technicians.

In addition, the needle biopsy is a highly invasive examination that usually involves inserting ten or more needles through the subject's perineum or rectum to collect a prostate tissue, also has a risk of complications and infectious diseases, and it is an examination with a problem of a decrease in the QOL of patients.

However, the percentage of subjects of the needle biopsy who have positive results (the percentage of subjects who are definitely diagnosed with prostate cancer) is not very high as described above.

One of reasons there of is that a considerable number of subjects with prostatic hyperplasia are included in subjects for which PSA values considered as the gray zone in the PSA screening, are detected because the total PSA value tends to increase even in patients with prostatic hyperplasia. Since the number of patients with prostatic hyperplasia is many times larger than the number of patients with prostate cancer, the range of the subjects for which the PSA values considered as the gray zone are detected is also large. As a result, the number of subjects undergoing the needle biopsy increases so that there is a possibility that a positive predictive value of prostate cancer decreases.

In addition, regarding the needle biopsy, in addition to the above-described cost and a burden on a subject, there are drawbacks that the probability of so-called false negatives, in which it is determined to be negative because no cancer cell is collected as a biopsy needle accidentally has no contact with prostate cancer tissues despite the presence of the cancer tissues (cells) in the subject's prostate, is estimated to be about 10 to 20%, and that there is a case where collected cancer tissues are biased on a site with malignancy lower than malignancy as a whole even when the biopsy needle reaches the cancer tissues, resulting in underestimation of malignancy. When considering these, there is also a problem that it is difficult to make an accurate diagnosis with one-time needle biopsy.

Meanwhile, under such circumstances, not only the PSA screening but also the "Gleason score" (GS) scored by tissue classifications such as cancer tissue morphology, infiltration stages, or growth patterns, or a grade group based on the Gleason score, an evaluation such as the TNM classification classified by stages, and further, an indicator such as risk classifications (NCCN classification and D'Amico classification) based on the above-described three evaluations (scores) are widely used now as a determination material to determine a treatment policy for prostate cancer. However, the PSA screening and needle biopsy described above are used in either method, and thus, it is difficult to completely eliminate the above-described drawbacks.

To solve this problem, markers with higher specificity than PSA, such as a marker that does not increase in prostatic hyperplasia, have been developed.

For example, in recent years, it has been found that properties of sugar chains in PSA change depending on affected diseases, in other words, the amount (percentage) of PSA having specific sugar chains changes in patients with prostate cancer. For example, Patent Literature 1 describes an invention in which the discrimination performance of prostate cancer is improved by observing the sugar chain structure of PSA.

In addition, Patent Literature 2 made by the present applicants describes an invention in which PSA having a specific sugar residue (which is abundant in PSA of a patient with prostate) at a terminal can be quickly detected with high sensitivity and high accuracy by an SPFS device.

In addition, the present applicants have obtained a patent on an invention relating to the acquisition of diagnostic information for improving the accuracy of discrimination between prostate cancer and benign prostate diseases (such as prostatic hyperplasia) by detecting a sugar chain on PSA and a sugar chain on total mucin (Patent Literature 3), and further, has filed a patent application for an invention relating to the acquisition of new diagnostic information for improving the accuracy of discrimination therebetween by detecting a sugar chain on PSA and a detection result of a kind of sugar chain contained in mucin in combination (Patent Literature 4).

In addition, the present inventors have published, through a paper, a result that about 40% of needle biopsies that would have been performed originally is likely to be avoidable by improving the accuracy of discrimination between prostate cancer and benign prostate diseases in PSA screening by increasing the number of clinical samples, acquiring information on sugar chains of PSA, and conducting studies based on the information. Patent Literature 5 discloses the use of PSA having an N-acetylgalactosamine (GalNac-PSA) residue as a marker for prostate cancers.

### (Non Patent Literature 1).

However, about 60% of people still undergo unnecessary needle biopsies although the number of patients undergoing unnecessary needle biopsies certainly becomes close to half in the technique disclosed in Non Patent Literature 1. Therefore, it can be said that the problems of economic and human loss and QOL of subjects as described above are still significant. Therefore it is necessary to more accurately determine a subject who should undergo the needle biopsy and a subject who does not need to undergo the needle biopsy.
Non Patent Literature 2 discloses the concept of using the overall value of PSA divided by the prostate volume (i.e. PSA without taking into account wether it has an N-acetylgalactosamine residue) to obtain the PSA-density.
Non-Patent Literature 3 discloses the usage of the concentration of PSA in serum as an early detection method of prostate cancer. In particular the document discloses a differentiation method of α2,6- and α2,3-sialylated isomers. The assay disclosed Non-Patent Literature 3 allows an quantitation of PSA glycoforms from urine and the differentiation between aggressive prostate camcers, indolent prostate cancers, and benign prostate hyperplasia.

### Citation List

### Patent Literature

Patent Literature 1: Japanese Patent No. 5754767
Patent Literature 2: Japanese Patent No. 5726038
Patent Literature 3: Japanese Patent No. 6323463
Patent Literature 4: Japanese Patent Application No. 2015-554444
Patent Literature 5: EP 3 358 352 A

### Non Patent Literature

Non Patent Literature 1: Int JMol Sci. 2017 Feb; 18(2): 261.
Non Patent Literature 2: NIALL M. CORCORAN ET AL: "The ability of prostate-specific antigen (PSA) density to predict an upgrade in Gleason score between initial prostate biopsy and prostatectomy diminishes with increasing tumour grade due to reduced PSA secretion per unit tumour volume : ABILITY OF PSA DENSITY TO PREDICT AN UPGRADE IN GLEASON SCORE", BJU INTERNATIONAL, vol. 110, no. 1, 1 July 2012 (2012-07-01), pages 36-42, XP055547746, ISSN: 1464-4096, DOI: 10.1111/j.1464-410X.2011.10681.x.
Non Patent Literature 3: GUINEVERE S. M. KAMMEIJER ET AL: "An In-Depth Glycosylation Assay for Urinary Prostate-Specific Antigen", ANALYTICAL CHEMISTRY, vol. 90, no. 7, 4 March 2018 (2018-03-04), pages 4414-4421, XP055675609, US, ISSN: 0003-2700, DOI: 10.1021/acs.analchem.7b04281.

### Summary of Invention

### Technical Problem

An object of the present invention is to provide a method for acquiring auxiliary information useful to assist a diagnosis or treatment of prostate cancer.

### Solution to Problem

The present inventors have found that a value of concentration of prostate specific antigen (GalNAc-PSA) per prostate volume (G-PSA density (G-PSAD)), obtained by dividing a concentration value of GalNAc-PSA having a β-N-acetylgalactosamine residue at a non-reducing terminal of a sugar chain by the prostate volume value, is useful as auxiliary information to assist the diagnosis or treatment of prostate cancer, and has completed the present invention.
[1] A method for acquiring auxiliary information to assist a diagnosis or treatment of prostate cancer, comprising athe following steps (A), (B), and (C),
   step (A): a step of acquiring a concentration value of a prostate specific antigen (GalNAc-PSA) having a β-N-acetylgalactosamine residue at a non-reducing terminal of a sugar chain contained in a sample derived from a living body,
   step (B): a step of acquiring a volume value of the prostate of the living body, and
   step (C): a step of dividing a the concentration value of a the prostate specific antigen (GalNAc-PSA) having a β-N-acetylgalactosamine residue at a non-reducing terminal of a sugar chain, contained in a sample derived from a living body, by athe volume value of the prostate of the living body to calculate a value (G-PSA density (G-PSAD)) of the concentration of the GalNAc-PSA per prostate volume, further comprising following steps (A) and (B),
   step (A): a step of acquiring the concentration value of the prostate specific antigen (GalNAc-PSA) having the β-N-acetylgalactosamine residue at the non-reducing terminal of the sugar chain contained in the sample derived from the living body, and
   step (B): a step of acquiring the volume value of the prostate of the living body wherein
   the step (A) is a step of acquiring the concentration value of the GalNAc-PSA by an interaction between a molecule having an affinity for the β-N-acetylgalactosamine residue and the GalNAc-PSA.
[2] The method for acquiring auxiliary information according to [1], further comprising
   step (D) of classifying calculated G-PSAD values into two or more groups according to magnitudes of the values.
[3] The method for acquiring auxiliary information according to [2], wherein
   the step (D) is a step of classifying the calculated G-PSAD values into three groups according to magnitudes of the values.
[4] The method for acquiring auxiliary information according to any one of [1] to [3], further comprising
   step (α) of acquiring a concentration value of a total prostate specific antigen (total PSA) contained in the sample derived from the living body.
[5] The method for acquiring auxiliary information according to any one of [1] to [4], wherein
   the concentration value of the total prostate specific antigen (total PSA) in the sample derived from the living body is more than 0 and equal to or less than 100 ng/ml.
[6] The method for acquiring auxiliary information according to any one of [1] to [4], wherein
   the concentration value of the total prostate specific antigen (total PSA) in the sample derived from the living body is 2 to 20 ng/ml.
[7] The method for acquiring auxiliary information according to [1], wherein
   the molecule having the affinity for the β-N-acetylgalactosamine residue is Wisteria floribunda lectin (WFA), soybean agglutinin (SBA), Vicia Villosa lectin (VVL), Trichosanthes japonica agglutinin-II (TJA-II), or an anti-β-N-acetylgalactosamine antibody.
[8] The method for acquiring auxiliary information according to [1] or [7], wherein
   the step (A) is a step of measuring the concentration value of the GalNAc-PSA by surface plasmon-field enhanced fluorescence spectroscopy.
[9] An auxiliary information acquisition system comprising: an input device; and an information processing device,
   wherein the input device is a device that inputs a concentration value of a GalNAc-PSA in a sample derived from a living body and a volume value of prostate of the living body, and
   the information processing device is a device that analyzes probability data for having prostate cancer based on information including a result obtained by dividing the concentration value of the GalNAc-PSA by the volume value of the prostate to calculate a value (G-PSA density (G-PSAD)) of the concentration of the GalNAc-PSA per prostate unit volume,
   the acquisition system performing the method for acquiring auxiliary information according to any one of [1] to [8].
[10]. A program that causes a computer to execute the acquisition method according to any one of [1] to [8].

### Advantageous Effects of Invention

According to the present invention, a medical worker other than a doctor such as a medical information technologist, a clinical engineer, and a clinical laboratory technician, can quickly acquire information to assist in the diagnosis or treatment of the prostate cancer with high accuracy, and the information can be provided to the doctor to assist the diagnosis or treatment. Further, the probability of being determined as a false positive can be reduced by using the information for screening, and a medical economic effect can be achieved and a burden on medical personnel and subjects can be decreased by reducing the number of subjects undergoing unnecessary needle biopsies.

### Brief Description of Drawings

Fig. 1A (three graphs on the left) is graphs illustrating values of total PSA (upper graph), GalNAc-PSA (middle graph), and G-PSAD (lower graph) in column plots, respectively, for patients with prostate cancer and patients with prostatic hyperplasia tested at Kyoto University.
   In addition, the three graphs on the right are ROC curves created based on the above results.
Fig. 1B is graphs illustrating results of similar tests with those in Fig. 1A conducted at Hirosaki University.
Fig. 2A is graphs illustrating correlations of the Gleason scores of patients with prostate cancer tested at Kyoto University with each of total PSA concentration values (upper graph), GalNAc-PSA concentration values (middle graph), and G-PSAD values (lower graph) in column plots (Example 3-1).
Fig. 2B is graphs illustrating correlations of the Gleason scores of the patients with prostate and patients with prostatic hyperplasia tested at Hirosaki University with each of values of total PSA (upper graph), GalNAc-PSA (middle graph), and G-PSAD (lower graph) in column plots (Example 3-2).
Fig. 3 is graphs illustrating correlations of a low group (low risk group), an intermediate group (intermediate risk group), and a high group (high risk group) according to the D'amico classification tested at Hirosaki University with each of values of total PSA (upper graph), GalNAc-PSA (middle graph), and G-PSAD (lower graph) in column plots.

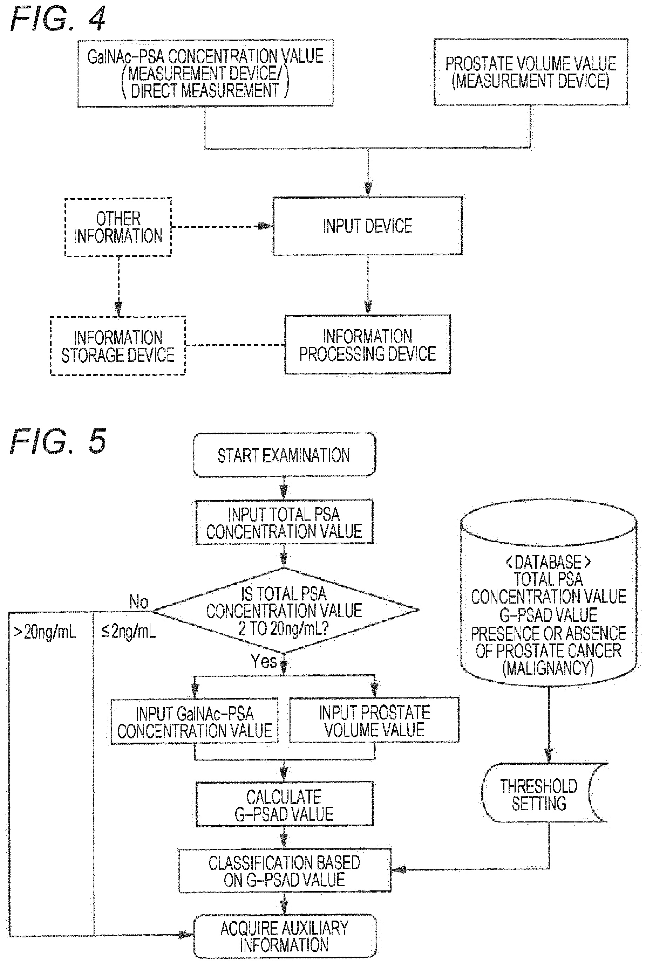

Fig. 4 is an example of a configuration diagram according to an auxiliary information acquisition system of the present invention.
Fig. 5 is a flowchart illustrating an aspect of the auxiliary information acquisition system of the present invention.

### Description of Embodiments

In a "method for acquiring auxiliary information" of the present invention, auxiliary information, used by reference to assist a diagnosis or treatment of prostate cancer, can be obtained by calculating a value (G-PSA density (G-PSAD)) of GalNAc-PSA concentration per prostate volume.

Specifically, as described above, the "auxiliary information" of the present invention can be referred to in the diagnosis of prostate cancer. Further, the "auxiliary information" of the present invention can be referred to in the process of assisting and treating the diagnosis of cancer malignancy in a patient for which a definitive diagnosis has been made. For example, in the case of determining whether the treatment (monitoring therapy, drug treatment, radiation treatment, or the like) performed to a patient with a confirmed diagnosis of prostate cancer is effective or the like, it is possible to determine the exacerbation, remission, recurrence, or the like of the cancer more accurately than a PSA test by referring to the "auxiliary information" of the present invention.

### <Method for Acquiring Auxiliary Information>

The method for acquiring auxiliary information of the present invention is a method for acquiring auxiliary information to assist a diagnosis or treatment of prostate cancer, and includes the following steps (A), (B) and (C).

Step (A): A step of acquiring a concentration value of a prostate specific antigen (GalNAc-PSA) having a β-N-acetylgalactosamine residue at a non-reducing terminal of a sugar chain contained in a sample derived from a living body.

Step (B): A step of acquiring a volume value of prostate of the living body.

Step (C): A step of dividing the concentration value of the prostate specific antigen (GalNAc-PSA) having the β-N-acetylgalactosamine residue at the non-reducing terminal of the sugar chain, contained in the sample derived from the living body, by the volume value of the prostate of the living body to calculate a value (G-PSA density (G-PSAD)) of the concentration of the GalNAc-PSA per prostate volume.

### <Step (A)>

The step (A) in the present invention is the step of acquiring the concentration value of the prostate specific antigen (GalNAc-PSA) having the β-N-acetylgalactosamine residue at the non-reducing terminal of the sugar chain contained in the sample derived from the living body, wherein the step (A) is a step of acquiring the concentration value of the GalNAc-PSA by an interaction between a molecule having an affinity for the β-N-acetylgalactosamine residue and the GalNAc-PSA.

The living body is not particularly limited, but is typically a human being, particularly a human being suspected of having prostate cancer. Examples of the sample derived from the living body include a liquid sample which can be collected by a non-invasive or minimally invasive method, such as blood, urine, and ascites collected from the living body. A serum prepared from whole blood that has been anticoagulated or plasma from which solid components such as blood cells have been removed by any method, such as centrifugation, is suitable as the sample in the present invention. In addition, the sample may be solid or semi-solid (viscous). In such a case, one prepared as a liquid having an appropriate viscosity by a known method or a diluent can also be used.

The amount of the sample used in the present invention is not particularly limited, but can be arbitrarily set depending on a method for measuring the sample, and is preferably 5 µL or more and 1,000 µL or less in general.

### <GalNAc-PSA>

In the present specification, GalNAc-PSA indicates a prostate specific antigen having a β-N-acetylgalactosamine residue (GalNAc residue) at a non-reducing terminal of a sugar chain. That is, any prostate specific antigen (GalNAc-PSA) having a β-N-acetylgalactosamine (GalNAc) residue at a non-reducing terminal of a sugar chain in PSA contained in the sample derived from the living body is included in "GalNAc-PSA" without being particularly limited since, for example, either PSA having a N-acetyl-D-galactosamine β1-4N acetylglucosamine (hereinafter referred to as LacdiNAc) residue (hereinafter referred to as LacdiNAc-PSA) or PSA having a β-N-acetylgalactosamine-galactosamine residue (GalNAc-Gal residue) or β-N-acetylgalactosamine-N-acetylglucosamine residue (GalNAc-GlcNAc residue (LacdiNAc)) has the GalNAc residue at the non-reducing terminal of the sugar chain that binds to the PSA.

A method for measuring a concentration value of GalNAc-PSA is not particularly limited, and various measurement methods, such as affinity column chromatography, mass spectrometry, enzyme immunoassay, and surface plasmon-field enhanced fluorescence spectroscopy (SPFS), can be used. From the viewpoint of accuracy of a measured concentration value, it is preferable to use the surface plasmon-field enhanced fluorescence spectroscopy.

A specific example thereof is a method of causing a sample to react with an anti-PSA antibody immobilized on a surface of a SPFS sensor and further causing GalNAc-PSA specifically bound to the anti-PSA antibody to react with a fluorescently labeled molecule that specifically binds to a GalNAc residue to produce a sandwich-type complex consisting of (anti-PSA antibody)...(GalNAc-PSA)...(fluorescently labeled molecule that specifically binds to GalNAc residue) ("..." indicates an antigen-antibody reaction or a lectin-glycoprotein reaction), and measuring the intensity of fluorescence used for labeling by SPFS.

The fluorescently labeled molecule that specifically binds to the GalNAc residue is not particularly limited, and examples thereof include a fluorescently labeled lectin that specifically binds to GalNAc, a fluorescently labeled anti-GalNAc antibody, and the like. As the fluorescently labeled anti-GalNAc antibody, an anti-β-N-acetylgalactosamine antibody, which is an antibody having a part or all of GalNAc as an epitope, can be used.

The "anti-PSA antibody" can be also produced by a general method, or a commercially available one can be purchased. From the viewpoint of measurement stability, it is preferable to use a monoclonal antibody rather than a polyclonal antibody. In addition, an antibody having a protein portion as an epitope rather than a sugar chain of PSA is preferable so as not to prevent the fluorescently labeled molecule that specifically binds to a specific sugar residue (β-GalNAc in the present invention) in a sugar chain from binding to the sugar residue.

Lectins that specifically bind to GalNAc are inexpensive and have excellent stability, and thus, are preferable as a molecule having an affinity for GalNAc, and any lectin may be used as long as the lectin has a sufficiently strong specific affinity for GalNAc. Specifically, lectins such as Wisteria floribunda lectin (WFA), soybean agglutinin (SBA), Vicia Villosa lectin (VVL), and Trichosanthes japonica agglutinin-II (TJA-II) can be exemplified, and can be separated (extracted) from organisms from which the lectins are derived, respectively, for example, seeds by a method such as affinity chromatography and purified, or commercially available products can be obtained.

Fluorescent labels for the lectin and anti-GalNAc antibody that specifically bind to GalNAc can be produced by binding a desired fluorescent substance using a general technique. In that case, a commercially available fluorescent substance labeling kit or the like can also be used. The fluorescent substance used for the fluorescent labeling of the lectin and the antibody can be appropriately selected from fluorescent dyes capable of emitting appropriate fluorescence according to a desired purpose.

### <Step (B)>

The step (B) in the present invention is the step of calculating the volume value of the prostate of the living body. The volume value may be obtained directly by a method such as palpation, catheterization, endoscopy, urethral pressure measurement, an X-ray examination, and CT scan, or may be calculated from a known formula by a method such as an MRI examination, transrectal ultrasonography, transrectal radial scanning, and transabdominal sector scanning.

The step (A) or (B) may be performed continuously, simultaneously, or independently by an independent device or operator. In the case of being performed independently, the step (A) may be performed first, or the step (B) may be performed first. Therefore, the acquired GalNAc-PSA concentration value and prostate volume value can be used in the next step (C) whether being acquired simultaneously or continuously, or acquired independently as long as being derived from the same living body.

### <Step (C)>

The step (C) in the present invention is the step of dividing the concentration value of the prostate specific antigen (GalNAc-PSA) having the β-N-acetylgalactosamine residue at the non-reducing terminal of the sugar chain, contained in the sample derived from the living body, by the volume value of the prostate of the living body to calculate the value (G-PSA density: GalNAc-PSA/Density (G-PSAD)) of the concentration of the GalNAc-PSA per prostate volume.

As described above, "GalNAc-PSA" is not particularly limited as long as being PSA in which the GalNAc residue is bound to the terminal end of PSA.

As a unit of the concentration value of "GalNAc-PSA", there is no particular unit like a SI basic unit defined in the international unit. For example, the following "U (unit)/ml" can be used as the unit, and "U (unit)/ml" is set based on the following criteria in the present specification, but other reference values may be used to set "U (unit)/ml".

In the present specification, a PSA protein having a sugar chain with which WFA lectin reacts as a standard antigen was dissolved in a buffer solution, a standard antigen solution was prepared to have a concentration of 1 ng/mL using an absorbance method (measured at wavelengths of 260 nm and 280 nm using a spectrophotometer U-3900 or the like), immunoassay (PSA test kit), or the like, and the concentration of GalNAc-PSA corresponding to a signal detected at the time of measuring the standard antigen solution under the same conditions as the method used for the sample was set to 1 U (unit)/ml.

Note that most of antigenic proteins are standard substances certified by national and international public institutions such as the National Metrology Institute of Japan (NMIJ) and the National Institute of Standards and Technology (NIST). However, the concentration unit is often set based on the original numerical value or criterion as described above in an antigen in which such a standard substance does not exist (for example, GalNAc-PSA in the present invention).

As another setting, a numerical value, obtained by converting a fluorescence signal (unit is count or digit), obtained from a detector (photomultiplier tube or photodiode) in which a standard antigen solution prepared to have a certain concentration is provided in an SPFS device produced by a known method under arbitrary reaction conditions, with a predetermined method can be also set as "U/mL". At this time, a device appropriately adjusted according to conditions such as an antigen to be used may be used as the SPFS device.

As yet another setting, samples are measured with a desired device at each institution for about 1000 healthy subjects, a measurement value at which 95% or more of numerical values of the healthy people fit is set as a reference value in a distribution graph or the like created from the obtained measurement values (for example, detected fluorescence intensity when the SPFS method is used), and it is possible to set a concentration value of GalNAc-PSA corresponding to the concentration of a standard antigen solution with which the same measurement value as the reference value is obtained, as "1 U/mL".

As a unit of volume, for example, cm³, cc, or ml can be used, and, for example, g/cm³, g/ml, g/cc, or the like can be used as a unit of density.

That is, a unit of the value of G-PSAD (GalNAc-PSA concentration value per prostate volume: G-PSA density) is not particularly limited, but for example, U/ml/cm³ can be adopted.

The GalNAc-PSA concentration value and the volume value of the prostate of the living body used in the step (C) can be obtained in the steps (A) and (B), respectively. In the present invention, the step (A), the step (B), and the step (C) may be performed by the same institution, the step (A), the step (B), and the step (C) may be performed by different institutions, or two steps among the above steps, may be performed in the same institution, and the remaining one step may be performed in another institution. In addition, the step (A) may be performed by a subject himself/herself.

For example, the G-PSAD value may be obtained by performing the step (C) by a research institution or an evaluation institution to which the GalNAc-PSA concentration value obtained in the step (A) performed by the subject and the volume value of the prostate of the living body obtained in the step (B) performed by a technician or the like in the hospital have been sent as data.

### <Step (D)>

A step (D) in the present invention is a step of classifying the G-PSAD value calculated by the step (C) into two or more groups according to the magnitude thereof. The number of groups to be classified is not particularly limited, but is preferably two or three groups, and is particularly preferably three groups from the viewpoint of ease of evaluation or analysis or from the viewpoint of determining a treatment method according to the malignancy.

### [Threshold-1]

In the method for acquiring auxiliary information to assist the diagnosis or treatment of prostate cancer of the present invention, it is preferable to set a threshold (cutoff value) for G-PSAD in order to use the obtained G-PSAD value more effectively as the auxiliary information to assist the diagnosis or treatment. For example, it is preferable to compare the calculated G-PSAD value of a subject (patient) with the threshold and classify the subject (patient) into two or more groups according to the magnitude in the step (D).

The threshold can be set in the same manner as a threshold for a diagnostic marker or a tumor marker, for example, using a general method similar to a known estimation method or information acquisition method based on the value of GalNAc-PSA concentration (G-PSAD) per prostate volume obtained in the step (C).

When setting the threshold, it is preferable to examine the correlation between the G-PSAD value and prostate cancer to create a database in advance, and set the threshold from the viewpoint of sensitivity (percentage of subjects with prostate cancer correctly determined as "positive" (%)) and specificity (percentage of subjects without prostate cancer correctly determined as "negative" (%)) based on the database.

For example, it is conceivable to prepare a database recording GalNAc-PSA concentration values, prostate volumes, and G-PSAD values contained in the respective samples (for example, sera) of a plurality of patients with prostate cancer (preferably patients with various stages of prostate cancer) and a plurality of people who have been diagnosed as not having prostate cancer (healthy people and/or patients with prostatic hyperplasia).

In general, it is understood from the database that the possibility of prostate cancer is high as the G-PSAD value increases. It is possible to set the threshold at which the desired accuracy (sensitivity, specificity) can be estimated by creating a box plot or a receiver operating characteristic curve (ROC curve) based on the G-PSAD values of the respective patients or subjects and whether the patients or subjects have been diagnosed with prostate cancer.

The sensitivity and specificity vary depending on a position where the threshold is set.

For example, if the threshold is set to be lower, the sensitivity increases (false negatives decrease), but false positives increase so that the specificity decreases. For example, when it is desired not to miss an affected patient as in a medical checkup, the threshold may be set to be low in this manner.

Conversely, if the threshold is set to be higher, the specificity increases (false positives decrease), but the sensitivity decreases. The threshold may be set to be higher in this manner, for example, when preferentially finding a subject having a high probability of being positive (affected) for subjects whose presence or absence of disease is difficult to determine in a preliminary diagnosis by measuring only the total PSA.

When carrying out the present invention, it is ideal to set, as a threshold, a value that makes the proportion of people with prostate cancer included in a range where the G-PSAD value exceeds the threshold as high as possible (that is, has high sensitivity) and allows people without prostate cancer not to be included in the range as much as possible (that is, has high specificity) in order to assist a positive determination (that is, determination that prostate cancer is present) that the subject has prostate cancer.

On the contrary, as a threshold to assist a negative determination regarding prostate cancer (that is, determination that prostate cancer is absent), it is ideal to set, as the threshold, a value that makes the proportion of people with prostate cancer included in a range where the G-PSAD value is below the threshold as low as possible and allows patients without prostate cancer to be included in the range as many as possible.

Specifically, when the unit of the value of G-PSAD is expressed in U/mL/cm³, it is preferable to set a threshold that can be used as information to assist the positive diagnosis that a subject has prostate cancer (that is, determination that the subject is a patient with prostate cancer) to 0.00146 to 0.0048 U/mL/cm³. In addition, when the sensitivity is prioritized over the specificity to prevent oversight (that is, when it is desired to set the sensitivity of 90% or more and the specificity of 78% or less), it is preferable to set the threshold to 0.00146 to 0.00212 U/mL/cm³. On the other hand, it is preferable to set a threshold to 0.00273 U/mL/cm³ or more when giving priority to the specificity (that is, when it is desired to set the sensitivity of 80% or less and the specificity of 86% or more) from the viewpoint of surely extracting a patient with prostate cancer.

Conversely, it is preferable to set a threshold that can be used as information to assist in a negative diagnosis regarding whether a subject has prostate cancer (that is, determination that the subject does not have prostate cancer) to 0.00146 U/mL/cm³ or less.

In addition, it is preferable to set a threshold to 0.00146 to 0.00324 U/mL/cm³ when the purpose is to obtain auxiliary information that is referred to for diagnosing that the probability of prostate cancer is low but follow-up observation is required, regarding the incidence of prostate cancer (that is, when it is desired to set the sensitivity of 70% or more and the specificity of 89% or less).

It is possible to acquire the auxiliary information that can be referred to for assisting the diagnosis that, for example, a patient with prostate cancer from which a sample has been collected has prostate cancer with a predetermined probability (sensitivity and specificity) if the G-PSAD value is equal to or more than the set threshold or that a patient does not have prostate cancer with a predetermined probability if the G-PSAD value is less than the threshold.

When the threshold is changed, the sensitivity and specificity change in tandem, and thus, it is desirable to perform adjustment (optimization) such that the both are balanced. As the number of samples, which is the population of measurement data, increases, it is possible to set a more reliable threshold.

### [Threshold-2]

In addition, it is possible to acquire auxiliary information that is referred to when diagnosing the malignancy of cancer in a patient with prostate cancer similarly by creating a database. The "malignancy" referred to here may be based on the Gleason score described above, may be based on a risk classification such as D'amico classification, or may be provided with a desired criterion.

For example, when the Gleason score is used as an evaluation criterion for the "malignancy", it is preferable to consider the Gleason score of 7 or higher (3+4 or 4+3 or higher) or (4+3 or higher) as "high malignancy", but other values, such as the Gleason score of 6 or higher (3+3), may be considered as "high malignancy". In addition, when the "malignancy" is evaluated using other criteria, for example, a total PSA concentration value of more than 10 ng/mL may be considered as "high malignancy", or a stage T2b or higher in the TNM classification may be considered as "high malignancy".

In this case, it is preferable to create a database by examining the correlation between the G-PSAD value and the malignancy of prostate cancer in advance and set a threshold based on the database when setting the threshold.

For example, it is conceivable to prepare a database recording GalNAc-PSA concentration values, prostate volumes, and G-PSAD values contained in the respective samples (for example, sera) taken from a plurality of patients with prostate cancer (preferably patients with prostate cancer of various degrees of malignancy).

In general, it is understood from the database that the malignancy of prostate cancer is high as the G-PSAD value increases. It is possible to set a threshold with which the estimation with the desired accuracy (sensitivity and specificity) can be performed by creating a box plot based on the G-PSAD value of each patient and the malignancy of the patient. For example, when the Gleason score is used as the malignancy as described above, it is possible to set the threshold with which the estimation with the desired accuracy (sensitivity and specificity) can be performed by creating the box plot based on the G-PSAD values of the respective patients and the respective Gleason scores thereof.

It is preferable to set a threshold, which can be used as the information to assist a positive diagnosis that a patient has prostate cancer and the malignancy of the prostate cancer is high (determination that the prostate cancer is likely to be high in malignancy requiring treatment), to 0.00408 U/mL/cm³ or more.

Conversely, it is preferable to set a threshold, which can be used as information to assist a positive diagnosis that a patient has prostate cancer but the malignancy of the prostate cancer is relatively low (determination that the malignancy is not so high that the prostate cancer requires immediate treatment, that is, the probability of having a malignancy sufficiently covered by follow-up observation of the progress (exacerbation, or the like) of cancer is high), to 0.00321 U/mL/cm³ or less.

For example, it is possible to acquire the auxiliary information that can be referred to for assisting the diagnosis that prostate cancer in a patient is likely to be highly malignant with a predetermined probability (sensitivity and specificity) if a G-PSAD value in a patient with prostate cancer is above the set threshold, or that the probability of having a low malignancy is high if the G-PSAD value is less than the threshold.

The sensitivity and specificity change in tandem when the threshold is changed similarly to the threshold related to the presence or absence of prostate cancer, and thus, it is desirable to perform adjustment (optimization) such that the both are balanced. As the number of samples (number of patients), which is the population of measurement data, increases, it is possible to set a more reliable threshold.

### <Step (a)>

A step (α) in the present invention is a step of measuring a concentration value of a total prostate specific antigen (total PSA) contained in a sample derived from a living body, and is the step performed using the same sample as the sample performed in the steps (A) to (C).

In the present invention, the concentration value of total prostate specific antigen (total PSA) contained in the sample derived from the living body is preferably more than 0 and 100 ng/ml or less, and is more preferably 2 to 20 ng/mL from the viewpoint as a criteria for patient selection. Specifically, the total PSA concentration value often takes a value of 2 to 20 ng/mL even for patients with benign prostatic disease (prostatic hyperplasia), which need to be distinguished from patients with prostate cancer, that is, often overlaps with a total PSA value of patients with prostate cancer in this range. Therefore, it can be said that the usefulness of the auxiliary information obtained by calculating the G-PSAD value is the best for patients with the total PSA concentration value of 2 to 20 ng/mL, which can be said to be a region where it is more difficult to distinguish between the patients with prostate cancer and patients with benign prostate diseases.

In addition, the total PSA can be used to set the threshold, configured to acquire the auxiliary information that can be referred to for assisting the diagnosis or treatment of prostate cancer of the present invention, as described above.

### <Acquisition System>

An auxiliary information acquisition system according to an embodiment of the present invention is a system configured to acquire auxiliary information, and is the system including an input device and an information processing device.

The input device is a device configured to input information for acquiring auxiliary information according to the present invention. Examples of the information include a concentration value of GalNAc-PSA and a volume value of prostate of a living body, and may further include a total PSA concentration value and a numerical value of the Gleason score.

For example, the input device is a receiving device that receives the GalNAc-PSA concentration value or the volume value of the prostate of the living body as digital data. In addition, the input device may be an input means that directly inputs the GalNAc-PSA concentration value or the volume value of the prostate of the living body, for example, a keyboard, a mouse, or a touch panel. When the GalNAc-PSA concentration value is measured by another independent device, it is preferable to provide the receiving device capable of receiving the value as digital data from the device.

In addition, the auxiliary information acquisition system according to the embodiment of the present invention may include a device capable of measuring the GalNAc-PSA concentration value, and preferably includes a SPFS device and a detector that can be used in correspondence with the device, for example.

The information processing device is a device that receives information acquired by the input device and acquires auxiliary information according to the present invention based on the information. Typically, the information processing device is a device that calculates a value (G-PSA density (G-PSAD)) of GalNAc-PSA concentration per prostate unit volume by calculating a value obtained by dividing the input GalNAc-PSA concentration value by the prostate volume value, and analyzes the information as needed. The information processed by the information processing device is preferably converted as digital data, may be subjected to arithmetic processing by a known means, or may be further processed into a graph or chart.

It is preferable that the information processing device include an information storage device configured to store information over time. For example, it is possible to observe the amount of change in a measurement value over time for a certain sample and a rate, a fluctuation, or the like of the change by storing data by associating basic data, such as an age, a weight, examination history, and the amount of change in each information with auxiliary information obtained at each time point for a subject from the input device or the like at a plurality of measurement points. In addition, it is possible to perform arbitrary analysis based on the above pieces of information, for example, creation of an ROC curve, measurement of AUC, or determination of a threshold based on those parameters.

### (Acquisition Program)

A program of the present invention describes instructions (processes) for the acquisition system of the present invention, and indicates a system program that controls each part of the acquisition system and processes data in order to execute the information processing or analysis by a central processing unit (CPU) of a computer

The program may be stored in the information processing device, or may be recorded in other computer-readable recording media, such as magnetic tapes (such as a digital data storage (DDS)), magnetic disks (such as a hard disk drive (HDD) and a flexible disk (FD)), optical disks (such as a compact disk (CD), a digital versatile disk (DVD), and a Blu-ray disk (BD)), magneto-optical disks (MO), flash memories (a solid state drive (SSD)), memory cards, USB memories, or the like, or may be provided independently.

### Examples

Next, the present invention will be described in more detail with reference to experimental examples, but the present invention is not limited thereto.

### [Production Example 1]

### <Production of Plasmon Excitation Sensor>

A trapezoidal prism transparent substrate made of resin was subjected to plasma washing to form a gold thin film on one side of the substrate by a sputtering method. A thickness of the gold thin film was 44 to 52 nm.

The substrate having the gold thin film formed thereon in this manner was immersed in an ethanol solution containing 1 mM of 10-carboxy-1-decanethiol for 20 minutes or more to form a self-assembled monolayer (SAM) formed of 10-carboxy-1-decanethiol on the surface of the gold thin film. The substrate was removed from this solution, washed with ethanol and isopropanol, and then, dried using an air gun.

A mixture, obtained by mixing 25 mM of MES-buffered saline, which contains 0.5 mM of N-hydroxysuccinimide (NHS), 0.5 mM of water-soluble carbodiimide (WSC), and 1 mg/mL of carboxymethyl dextran (CMD) (Meito Sangyo Co., Ltd.; CMD-500-06I4: average molecular weight 500,000, degree of substitution 0.51), and 10 mM of NaCl solution (pH 6.0) each by 0.8 mL, was dropped to the dried substrate to cause reaction for 20 minutes, thereby forming a CMD film on the SAM on the substrate. Further, a sealing material having a thickness of 100 µm was placed on the CMD film to form a flow path having a height of 100 µm, thereby producing a plasmon excitation sensor.

### [Production Example 2]

### <Preparation of Anti-PSA Monoclonal Antibody-Immobilized Substrate>

A surface of a plasmon excitation sensor prepared in Production Example 1 was washed with MES-buffered saline to equilibrate the surface of the plasmon excitation sensor.

Subsequently, 5 mL of MES-buffered saline containing 50 mM of N-hydroxysuccinimide (NHS) and 100 mM of water-soluble carbodiimide (WSC) was caused to react on the surface of the plasmon excitation sensor for 20 minutes. Further, 20 µL of anti-PSA monoclonal antibody solution (Mikuri Immunology Research Institute Co., Ltd.; 50 µg/mL) was caused to react for 30 minutes to bind the antibody to CMD on the plasmon excitation sensor, thereby preparing a resultant having an anti-PSA monoclonal antibody-immobilized CMD film (referred to as a measurement region) on the plasmon excitation sensor.

Then, blocking was performed for 10 minutes using phosphate-buffered saline (PBS) containing 1 wt% of bovine serum albumin (BSA) in order to perform non-specific adsorption prevention treatment in the flow path.

### [Production Example 3]

### <Production of Fluorescently Labeled Lectin (Alexa Fluor (registered trademark) 647 Labeled WFA)>

Wisteria floribunda lectin (WFA: VECTOR Laboratories inc.: L-1350) equivalent to 100 µg and a fluorescent substance labeling kit "Alexa Fluor (registered trademark) 647 protein labeling kit" (Thermo Fisher Scientific Co., Ltd.) were used to produce Alexa Fluor (registered trademark) 647-labeled WFA (hereinafter referred to as fluorescently labeled lectin in the present experimental example) by the following method.

The above WFA was mixed with 0.1 M of sodium bicarbonate and Alexa Fluor 647 reactive dye included in the above kit and allowed to react at room temperature for 1 hour. Then, the resultant was subjected to gel filtration chromatography and ultrafiltration to remove impurities such as Alexa Fluor 647 reactive dye, which were not used for labeling, thereby obtaining a fluorescently labeled WFA lectin. Then, the absorbance was measured to quantify the concentration of the fluorescently labeled lectin.

### [Experimental Example 1-1]

At Kyoto University, a national university corporation, verification was performed for sera of 160 cases of patient with prostate cancer diagnosed by prostate needle biopsies and 51 cases of patient with prostatic hyperplasia (total: 211 cases). The histopathological diagnosis of each patient was determined by the Gleason score.

Table 1 shows total PSA concentration values, GalNAc-PSA (LacdiNAc-PSA) values, values (G-PSAD) of GalNAc-PSA per prostate volume, and the Gleason scores in each group of patients.

Note that 211 people who are considered as patient background have the total PSA of 2 to 20 ng/mL and represent the number of subjects who have undergone needle biopsies, of which 160 people (75.8%) were diagnosed as patients with prostate cancer and 51 people (24.2%) were diagnosed with prostatic hyperplasia.

From each group, a total PSA concentration (ng/mL), a GalNAc-PSA concentration (U/ml), and a G-PSAD value (U/ml/cm³) were measured and calculated, and the respective numerical values are illustrated in the upper column of the following Table 1.

In addition, 144 patients diagnosed with prostate cancer underwent radical prostatectomy. The Gleason scores in resected prostate cancer tissues were classified. The number of patients (number of samples) corresponding to each score is illustrated in the lower column of Table 1.

**[Table 1]**

| Patient background | | | |
|---|---|---|---|
| n = 211 | Patient With Prostate Cancer (n = 160) | Patient With Prostatic Hyperplasia (n = 51) | |
| | median (range) | median (range) | P value |
| Total PSA (ng/mL) | 7.59 (0.86-4076) | 6.51 (0.95-18.5) | 0.0723 |
| GalNAc-PS (U/mL) | 0.149 (0.019-53.609) | 0.083 (0.025-0.225) | < 0.0001 |
| Prostate Volume (cm³) | 27.0 (8.60-77.0) | 30.5 (8.00-120) | < 0.0001 |
| PSA-GiD (U/mL/cm³) | 0.00509 (0.00066-1.787) | 0.00154 (0.00027-0.0064) | < 0.0001 |

| Gleason Score | n = 144 | | |
|---|---|---|---|
| 6 (3+3) | 20 | | |
| 7 (3+4) | 68 | | |
| 7 (4+3) | 38 | | |
| 8 (4+4) | 9 | | |
| 8 (3+5) | 4 | | |
| 9 (4+5) | 5 | | |

The total PSA concentration value was quantified according to the manual using the prostate specific antigen kit "Total PSAAbbott" and the "ARCHITECT Analyzer i1000SR" system (each of which is manufactured by Abbott Japan Co., Ltd.).

The quantification of GalNAc-PSA was performed by the following method using a flow path type SPFS measuring member provided with the anti-PSA monoclonal antibody-immobilized substrate prepared in Production Examples 1 and 2 and the fluorescently labeled WFA prepared in Production Example 3.

100 µL of a diluted solution was added to 20 µL of a serum sample, and the mixture was well stirred in a tube to prepare a mixed solution. 100 µL of this mixed solution was circulated and sent to the flow path of the flow path type SPFS measuring member to react with the measurement region on the measuring member for 30 minutes. Then, TBS (TBS-T) containing 0.05% by weight of Tween (registered trademark) 20 was sent and washed for 3 minutes. Subsequently, 100 µL of the Alexa Fluor 647-labeled WFA solution (WFA concentration: 10 µg/mL) prepared in Production Example 3 was circulated and sent to the flow path to react with the measurement region for 10 minutes. Then, TBS-T was sent again and washed for 5 minutes. Further, in a state where the flow path is filled with TBS-T, excitation light of Alexa Fluor 647 was emitted using a laser beam having a wavelength of 647 nm, and the measured fluorescence intensity (fluorescence signal) was measured.

The measured fluorescence intensity of each test sample was converted into a GalNAc-PSA concentration value based on a calibration curve prepared using a test sample having a known GalNAc-PSA value.

Regarding prostate volume information, a prostate volume was measured by transrectal ultrasonography (TRUS) using a device such as Aloka prosound alpha7 (Hitachi, Ltd.), which is an ultrasonic diagnostic device, or magnetic resonance imaging (MRI) using a device such as Signa HDx (GE Helthcare) which is a 3D high-quality MRI device. The value (G-PSAD) of GalNAc-PSA per prostate volume was calculated by dividing the GalNAc-PSA concentration value by the measured prostate volume.

### <Results and Discussions>

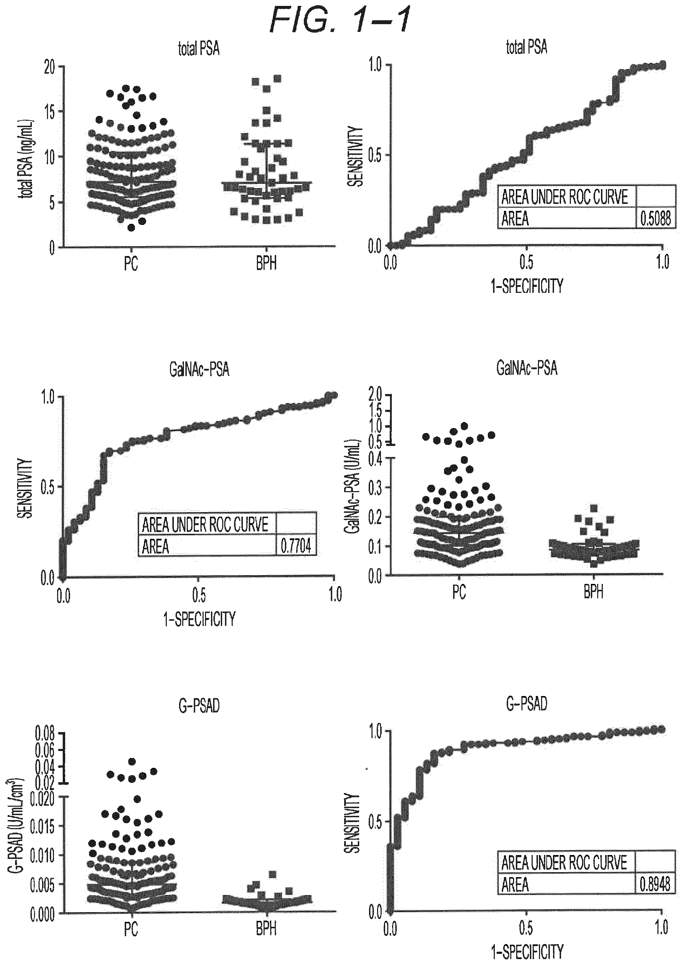

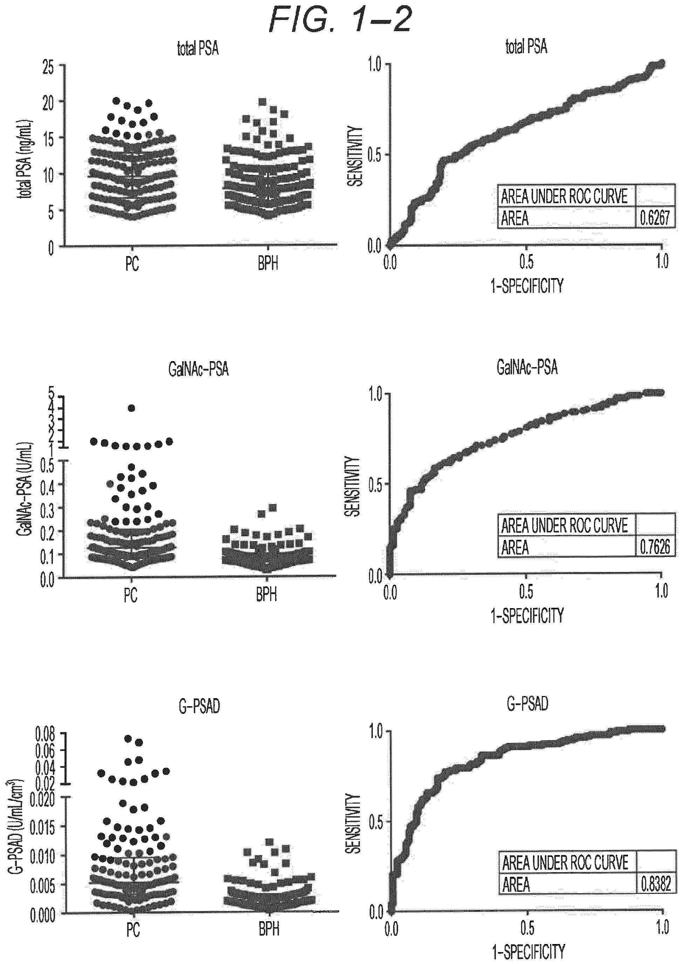

For the above-described patients with prostate cancer and subjects with prostatic hyperplasia (prostate cancer: 145 cases, prostatic hyperplasia: 47 cases), the total PSA concentration values, the GalNAc-PSA concentration values, and the G-PSAD values are illustrated in column plots (on the left in Fig. 1A). In addition, a relative operating characteristic curve (ROC curve) was created based on each result (on the right in Fig. 1A).

The following analysis was performed based on the column plots and ROC curves created based on the measured numerical values.

The area under the curve (AUC) was measured for each group based on the ROC curve created above. In general, it is determined that the predictive performance and diagnostic performance for a target disease are high as the AUC value is high.

The AUC value based on the ROC curve created based on the G-PSAD value calculated in the step (C) of the method for acquiring auxiliary information of the present invention was 0.8948. On the other hand, a measurement value of AUC based on a conventional total PSA concentration value was 0.5080, and a measurement value of AUC based on the GalNAc-PSA concentration value alone without adding the prostate volume information was 0.7704.

It is understood that the diagnostic performance using the G-PSAD value shows clearly excellent results as compared with the conventional diagnostic performance based on the total PSA and GalNAc-PSA concentration values. It is said that an excellent test is performed when the AUC value of the ROC curve exceeds 0.8 as an index of general diagnostic performance, but the AUC value of the ROC curve using the G-PSAD value greatly exceeds 0.8. Therefore, it was found that the test using the G-PSAD value is extremely useful.

### [Experimental Example 1-2]

An experiment similar to Experimental Example 1-1 was conducted at Hirosaki University which is a national university corporation. Table 2 shows total PSA concentration values, GalNAc-PSA concentration values, and GalNAc-PSA concentration values per prostate volume (G-PSAD values) in each group of patients, and the Gleason scores and the D'Amico risk classification.

Note that 339 people who are considered as patient background have the total PSA of 2 to 20 ng/mL and represent the number of subjects who have undergone needle biopsies, of which 150 people (44.2%) were diagnosed as patients with prostate cancer and 189 people (55.8%) were diagnosed with prostatic hyperplasia. 134 people having been risk-classified based on the results of needle biopsies or the like were classified into a high-risk group, a medium-risk group, and a low-risk group based on the D'Amico risk classification, and results thereof are illustrated in the lower column of Table 2.

In addition, 124 patients diagnosed with prostate cancer underwent radical prostatectomy. The Gleason scores in resected prostate cancer tissues were classified. The number of patients (number of samples) corresponding to each score is illustrated in the middle column of Table 2.

**[Table 2]**

| Patient background | | | |
|---|---|---|---|
| n = 339 | Patient With Prostate Cancer (n = 150) | Patient With Prostatic Hyperplasia (n = 169) | |
| | median (range) | median (range) | P value |
| Total PSA (ng/mL) | 9.93 (4.01-19.99) | 7.50 (4.06-19.69) | < 0.0001 |
| GalNAc-PS (U/mL) | 0.180 (0.014-2.43) | 0.067 (0.001-0.387) | < 0.0001 |
| Prostate Volume (cm³) | 24.4 (7.71-179) | 37.4 (11.0-147) | < 0.0001 |
| PSA-GiD (U/mL/cm³) | 0.00716 (0.00066-0.177) | 0.00168 (0.00002-0.0049) | < 0.0001 |

| Gleason Score | n = 124 | | |
|---|---|---|---|
| 6 | 3 | | |
| 7 | 38 | | |
| 8 | 16 | | |
| 9 | 64 | | |
| 10 | 3 | | |

| D'amico Risk Classification | n = 134 | | |
|---|---|---|---|
| Low | 8 | | |
| Intermediate | 61 | | |
| High | 65 | | |

The measurement of the total PSA concentration value and the GalNAc-PSA concentration value, and the calculation of G-PSAD were performed by the same methods as those in Example 1-1.

For the above-described patients with prostate cancer and subjects with prostatic hyperplasia (prostate cancer: 150 cases, prostatic hyperplasia: 189 cases), the total PSA concentration values, the GalNAc-PSA concentration values, and the G-PSAD values are illustrated in column plots (on the left in Fig. 1B). In addition, a relative operating characteristic curve (ROC curve) was created based on each result (on the right in Fig. 1B).

The same analysis as in Example 1-1 was performed based on the column plots and ROC curves created based on the measured numerical values.

The AUC value based on the ROC curve created based on the G-PSAD value calculated in the step (C) of the method for acquiring auxiliary information of the present invention was 0.8382. On the other hand, a conventionally measured value of AUC based on the total PSA concentration value was 0.6267, and a measurement value of AUC based on the GalNAc-PSA concentration value alone without adding the prostate volume information was 0.7626.

Even in the results of Hirosaki University, the diagnostic performance of G-PSAD shows clearly excellent results as compared with the diagnostic performance based on the GalNAc-PSA concentration value. As described above, it is said to be an excellent test if the AUC value of ROC analysis exceeds 0.8. In the present experimental example, it was found to be an extremely useful test since even the number of cases exceeding 300 met the criteria.

That is, it is understood that the auxiliary information acquired in the present invention is more excellent in terms of the sensitivity and specificity than the conventional information used for the diagnosis and determination using the total PSA concentration value or using the GalNAc-PSA concentration value. Further, the above results have been obtained using the sample derived from the patient for which it is difficult to determine the positive or negative and of which the total PSA concentration value is a relatively low value of 20 ng/mL or less (mostly 10 ng/mL or less). Thus, it has been found that the auxiliary information acquired in the present invention is useful as the auxiliary information in the scene of the diagnosis and treatment with higher accuracy than the information obtained by the conventional method.

### [Experimental Example 2]

In the experiment conducted at Kyoto University (Experimental Example 1-1), the proportion (%) of correct determination on subjects with prostate cancer as "positive" is defined as "sensitivity", the proportion (%) of correct determination on subjects without prostate cancer as "negative" is defined as "specificity", and thresholds set based on each of the sensitivity and specificity are shown in Tables 3-1 to 3-3.

**[Table 3-1]**

| Sensitivity and Specificity of Cancer/Non-cancer Determining Performance and Thresholds based on Sensitivity and Specificity using Total PSA Concentration Value as Auxiliary Information | | |
|---|---|---|
| Sensitivity (%) | Specificity (%) | Threshold (ng/ml) |
| 95 | 15 | > 3.950 |
| 90 | 17 | > 4.320 |
| 80 | 19 | > 5.125 |
| 70 | 28 | > 5.785 |
| 60 | 47 | > 6.535 |
| 50 | 51 | > 7.165 |

**[Table 3-2]**

| Sensitivity and Specificity of Cancer/Non-cancer Determining Performance and Thresholds based on Sensitivity and Specificity using GalNAc-PSA Concentration Value as Auxiliary Information | | |
|---|---|---|
| Sensitivity (%) | Specificity (%) | Threshold (U/ml) |
| 95 | 6 | > 0.0570 |
| 90 | 28 | > 0.0725 |
| 81 | 62 | > 0.0915 |
| 70 | 83 | > 0.1085 |
| 61 | 85 | > 0.1215 |
| 50 | 87 | > 0.1445 |

**[Table 3-3]**

| Sensitivity and Specificity of Cancer/Non-cancer Determining Performance and Thresholds based on Sensitivity and Specificity using G-PSAD Value as Auxiliary Information | | |
|---|---|---|
| Sensitivity (%) | Specificity (%) | Threshold (U/ml/cm³) |
| 95 | 41 | > 0.00146 |
| 90 | 78 | > 0.00212 |
| 80 | 86 | > 0.00273 |
| 70 | 89 | > 0.00324 |
| 60 | 95 | > 0.00408 |
| 50 | 97 | > 0.00480 |

### <Discussion>

Regarding those using the G-PSAD value as the auxiliary information, it has been found that "specificity", that is, the proportion at which people without cancer were correctly determined as "negative" was significantly higher than the others with the same sensitivity, that is, a numerical value obtained by correctly determining people with cancer as "positive" at each proportion. In other words, the proportion of false negatives in the patients who have been determined as positive for prostate cancer is reduced, and therefore, the auxiliary information is likely to have the high accuracy.

In general, it is preferable to select a threshold with sensitivity as high as possible at a screening stage (pre-stage of histopathological diagnosis (needle biopsy)) (with a low possibility of missing cancer (false negative)) and high specificity (that is, capable of avoiding an unnecessary needle biopsy).

When the G-PSAD value is used as the auxiliary information, the threshold of 0.00212 (sensitivity 90%, specificity 78%) can be selected as one index. In other words, this threshold illustrates a possibility that about 80% of unnecessary needle biopsies can be avoided.

In addition, when the threshold of G-PSAD is set to 0.00212 as will be described later, it can be estimated that the sensitivity is higher than 95% and the specificity is less than 45% (see Table 4-3). Therefore, it can be said that the G-PSAD value is useful for more accurate determination of the malignancy of prostate cancer according to the presence or absence of prostate cancer, and the information on G-PSAD is considered to be extremely useful in this respect as well.

### [Experimental Example 3-1]

### <Determination on Malignancy of Prostate Cancer Using Total PSA Concentration Value, GalNAc-PSA Concentration Value, And G-PSAD Value as Auxiliary Information>

Next, a comparative study was conducted on the effectiveness of determination on the malignancy of prostate cancer in the case of using the total PSA concentration value, the GalNAc-PSA concentration value, and the G-PSAD value as the auxiliary information.

At Hirosaki University, a national university corporation, sera, collected from 124 patients with prostate cancer for which the malignancy was determined by Gleason score determination with the radical prostatectomy were used as samples.

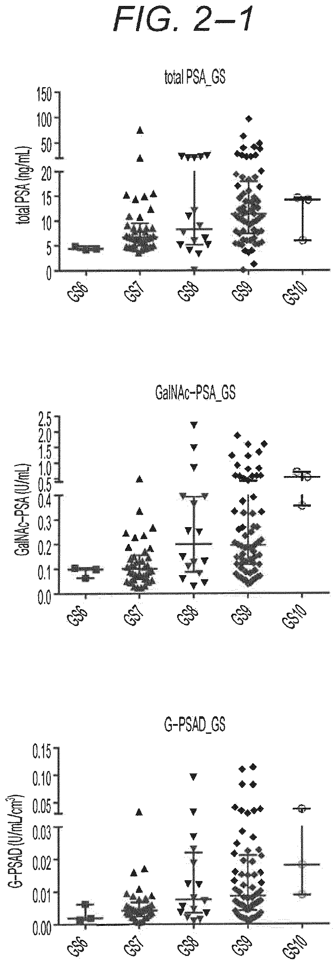

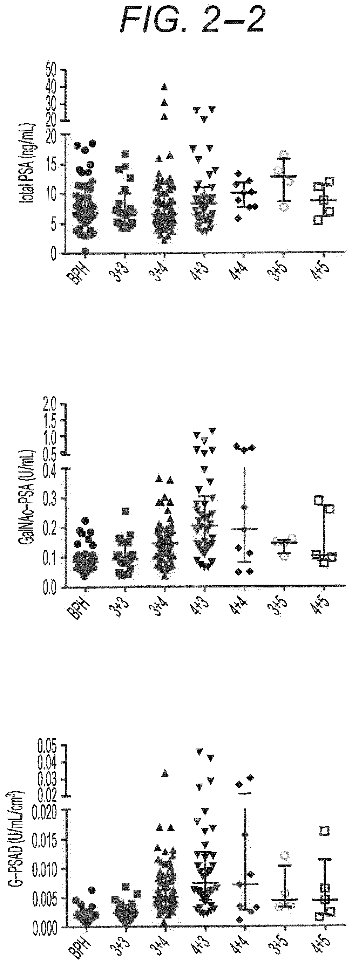

Measurement results of various markers are illustrated in column plots in which the respective values of the total PSA concentration value (upper graph), the GalNAc-PSA concentration value (middle graph), and the G-PSAD value (lower graph) obtained as above are plotted for each Gleason score (Fig. 2A). The total PSA concentration value, the GalNAc-PSA concentration value, and the G-PSAD value were calculated by the same methods as those in Experimental Example 1-1. Although the number of cases is small, a difference was observed most in distributions of groups with the Gleason score of 6 and 7 or higher in G-PSAD, and it was confirmed that the value increases along with the increase of the Gleason score.

### [Experimental Example 3-2]

Further, the same experiment as that in Experimental Example 3-1 was performed using sera, collected from patients with prostate cancer for which the Gleason score were determined by radical prostatectomy at Kyoto University, a national university corporation, (in this experimental example, the Gleason score is expressed as a sum of a dominant lesion and an associated lesion) and 51 patients with prostatic hyperplasia (BPH), as samples. Fig. 2B illustrates the column plot in which the obtained results are plotted for prostatic hyperplasia or each Gleason score.

### <Results and Discussions>

In Examples 3-1 and 3-2 above, it has been found that there is a strong correlation between the G-PSAD value and the malignancy of prostate cancer (Gleason score) through the studies at two different centers.

As illustrated in the lower graph in Fig. 2B, it has been confirmed that a case group with a relatively low malignancy, of which the Gleason score is 3+3 in G-PSAD, almost overlaps with the distribution of a prostatic hyperplasia group, and indicates a clearly lower value as compared with distributions of groups with the Gleason score of 3+4 and 4+3 (GS = 7) or higher. This result means that it is possible to predict highly malignant cancer that should be actively treated using the index called G-PSAD with high probability without performing a prostate needle biopsy.

From the above results, it can be said that it is possible to grasp a disease state (cancer malignancy) more accurately by using the G-PSAD value, and selection of an appropriate treatment method can greatly contribute to improvement in the prognosis and QOL of patients.

Note that a patient with the Gleason score of 3+4 or 4+3 (that is, the sum of Gleason scores is 7 or more) is usually classified as a patient who requires treatment such as surgery (surgical treatment), radiation treatment, endocrine therapy (hormone therapy), and chemotherapy.

For example, GS 3+3 is considered as a group having cancer but is less likely to die directly with the cancer even if active treatment is not performed in many cases. For some patients in this group, a treatment method called follow-up observation (active surveillance: monitoring therapy) is selected. Currently, whether a target to which monitoring therapy is applicable is determined based on results of a PSA test and a prostate biopsy (Gleason score). However, there is a case where the monitoring therapy based on incorrect prediction of malignancy is selected due to a fact that it is difficult to predict the malignancy by the PSA test, a sampling error of the prostate needle biopsy (such as a biopsy needle not sticking to major cancer tissues), or the like. There is a drawback that invasive tests such as regular PSA tests and prostate re-biopsies are required to safely perform the monitoring therapy.

In addition, although the monitoring therapy is less invasive than surgery, an annual prostate needle biopsy is required, and patients are constantly exposed to anxiety about when their tumors will become malignant. If there is a marker that predicts the presence of highly malignant prostate cancer in such patients with an extremely high probability as described above, not only the accuracy regarding the selection of the monitoring therapy is improved, but also a disease condition can be more accurately grasped by measuring the G-PSAD value during the follow-up observation, and as a result, the patient's anxiety can be alleviated.

### [Experimental Example 4]

In Example 2, the proportion (%) of correctly determination on subjects with prostate cancer as "positive" is defined as "sensitivity", and the proportion of correct determination on subjects without prostate cancer as "negative" is defined as "specificity". In Experimental Example 4, the proportion (%) of correct determination on people with highly malignant cancer as "positive" is defined as "sensitivity", the proportion of correct determination on people without highly malignant cancer as "negative" is defined as "specificity", and thresholds set based on each of the sensitivity and specificity are shown in Tables 4-1 to 4-3 similarly to Example 2.

**[Table 4-1]**

| Sensitivity and Specificity of Highly Malignant Prostate Cancer and Thresholds based on Sensitivity and Specificity using Total PSA Concentration Value as Auxiliary Information | | |
|---|---|---|
| Sensitivity (%) | Specificity (%) | Threshold (ng/ml) |
| 95 | 0 | > 3.685 |
| 90 | 15 | > 4.475 |
| 80 | 35 | > 5.410 |
| 70 | 35 | > 5.805 |
| 60 | 40 | > 6.560 |
| 50 | 70 | > 7.590 |

**[Table 4-2]**

| Sensitivity and Specificity of Predicting Performance of Highly Malignant Prostate Cancer and Thresholds based on Sensitivity and Specificity using GalNAc-PSA Concentration Value as Auxiliary Information | | |
|---|---|---|
| Sensitivity (%) | Specificity (%) | Threshold (U/ml) |
| 95 | 20 | > 0.0660 |
| 90 | 20 | > 0.0745 |
| 80 | 60 | > 0.1055 |
| 70 | 75 | > 0.1195 |
| 60 | 75 | > 0.1380 |
| 50 | 80 | > 0.1505 |

**[Table 4-3]**

| Sensitivity and Specificity of Predicting Performance of Highly Malignant Prostate Cancer and Thresholds based on Sensitivity and Specificity using G-PSAD Value as Auxiliary Information | | |
|---|---|---|
| Sensitivity (%) | Specificity (%) | Threshold (U/ml/cm³) |
| 95 | 45 | > 0.00218 |
| 90 | 55 | > 0.00260 |
| 81 | 70 | > 0.00321 |
| 70 | 85 | > 0.00408 |
| 60 | 90 | > 0.00480 |
| 50 | 95 | > 0.00568 |

### <Discussion>

Regarding those using the G-PSAD value as auxiliary information, it has been found that, when the value of sensitivity of 90%, for example is set as the threshold and a subject with a value higher than the threshold is considered as "positive", the "specificity" in subjects indicating a value lower than the G-PSAD value is significantly higher as compared with the case where the other two values are used as the auxiliary information. In other words, when the G-PSAD value is used as the auxiliary information, the proportion of false negatives (erroneously determined as highly malignant prostate cancer) is lower than those in the other cases. Therefore, it can be said that G-PSAD can serve as the auxiliary information for highly accurate prediction of malignancy.

### [Experimental Example 5]

As described above, in recent years, the risk classification, such as the D'Amico classification, which is a complex combination of TNM classification based on stages, PSA values, histopathological diagnosis (Gleason score grade group), has been widely used. A comparative study was conducted on the correlation with the D'Amico classification when each value of the total PSA, GalNAc-PSA, and G-PSAD was used as auxiliary information.

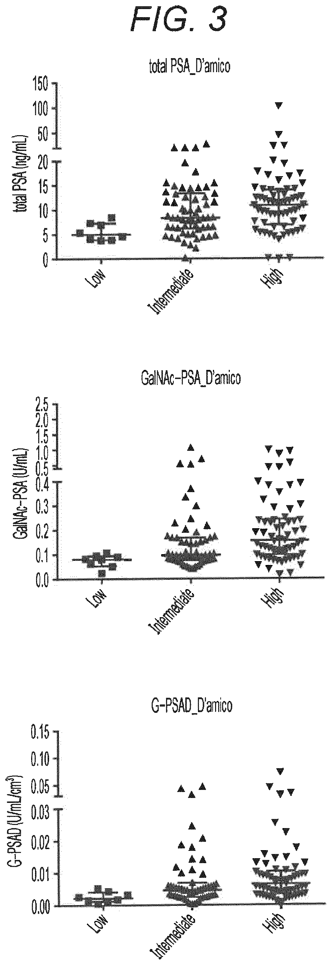

At Hirosaki University, a national university corporation, sera of patients with prostate cancer classified by the D'amico classification were used as samples. Fig. 3 illustrates correlation results with the D'amico classification in the case of using each value of the total PSA (upper graph), GalNAc-PSA (middle graph), and G-PSAD (lower graph) obtained as above as the auxiliary information.

The correlation was confirmed between the result of D'Amico classification and the result of using the G-PSAD value as the auxiliary information. It has been suggested that the risk classification can be predicted with a high probability without actually performing the histopathological diagnosis particularly for an intermediate group (medium-risk group) and a high group (high-risk group) in the D'Amico classification when the G-PSAD value is used as the auxiliary information as compared with the total PSA concentration value and GalNAc-PSA concentration value.

## Claims

1. A method for acquiring auxiliary information to assist a diagnosis or treatment of prostate cancer, comprising the following steps (A), (B), and (C),
step (A): a step of acquiring a concentration value of a prostate specific antigen (GalNAc-PSA) having a β-N-acetylgalactosamine residue at a non-reducing terminal of a sugar chain contained in a sample derived from a living body,
step (B): a step of acquiring a volume value of the prostate of the living body, and
step (C): a step of dividing the concentration value of the GalNAc-PSA by the volume value of the prostate of the living body to calculate a value (G-PSA density (G-PSAD)) of the concentration of the GalNAc-PSA per prostate volume,
wherein
the step (A) is a step of acquiring the concentration value of the GalNAc-PSA by an interaction between a molecule having an affinity for the β-N-acetylgalactosamine residue and the GalNAc-PSA.

2. The method for acquiring auxiliary information according to claim 1, further comprising step (D) of classifying calculated G-PSAD values into two or more groups according to magnitudes of the values.

3. The method for acquiring auxiliary information according to claim 2, wherein
the step (D) is a step of classifying the calculated G-PSAD values into three groups according to magnitudes of the values.

4. The method for acquiring auxiliary information according to any one of claims 1 to 3, further comprising step (α) of acquiring a concentration value of a total prostate specific antigen (total PSA) contained in the sample derived from the living body.

5. The method for acquiring auxiliary information according to any one of claims 1 to 4, wherein
the concentration value of the total prostate specific antigen (total PSA) in the sample derived from the living body is more than 0 and equal to or less than 100 ng/ml.

6. The method for acquiring auxiliary information according to any one of claims 1 to 4, wherein
the concentration value of the total prostate specific antigen (total PSA) in the sample derived from the living body is 2 to 20 ng/ml.

7. The method for acquiring auxiliary information according to claim 1, wherein
the molecule having the affinity for the β-N-acetylgalactosamine residue is Wisteria floribunda lectin (WFA), soybean agglutinin (SBA), Vicia Villosa lectin (VVL), Trichosanthes japonica agglutinin-II (TJA-II), or an anti-β-N-acetylgalactosamine antibody.

8. The method for acquiring auxiliary information according to claim 1 or 7, wherein
the step (A) is a step of measuring the concentration value of the GalNAc-PSA by surface plasmon-field enhanced fluorescence spectroscopy.

9. An auxiliary information acquisition system comprising: an input device; and an information processing device,
wherein the input device is a device that inputs a concentration value of a GalNAc-PSA in a sample derived from a living body and a volume value of prostate of the living body, and
the information processing device is a device that analyzes probability data for having prostate cancer based on information including a result obtained by dividing the concentration value of the GalNAc-PSA by the volume value of the prostate to calculate a value (G-PSA density (G-PSAD)) of the concentration of the GalNAc-PSA per prostate unit volume, the acquisition system performing the method for acquiring auxiliary information according to any one of claims 1 to 8.

10. A program that causes a computer to execute the acquisition method according to any one of claims 1 to 8.

## Patentansprüche

1. Verfahren zur Gewinnung von Zusatzinformationen zur Unterstützung einer Diagnose oder Behandlung von Prostatakrebs, umfassend die folgenden Schritte (A), (B) und (C),
Schritt (A): ein Schritt der Erfassung eines Konzentrationswertes eines prostataspezifischen Antigens (GalNAc-PSA) mit einem β-N-Acetylgalactosamin-Rest an einem nicht-reduzierenden Ende einer Zuckerkette, das in einer von einem Organismus stammenden Probe enthalten ist,
Schritt (B): ein Schritt der Erfassung eines Volumenwertes der Prostata des Organismus, und
Schritt (C): ein Schritt des Teilens des Konzentrationswertes des GaINAc-PSA durch den Volumenwert der Prostata des Organismus, um einen Wert (G-PSA-Dichte (G-PSAD)) der Konzentration des GaINAc-PSA pro Prostatavolumen zu berechnen,
wobei
der Schritt (A) der Erfassung des Konzentrationswertes des GaINAc-PSA durch eine Wechselwirkung zwischen einem Molekül mit einer Affinität für den β-N-Acetylgalactosamin-Rest und dem GaINAc-PSA erfolgt.

2. Verfahren zur Gewinnung von Zusatzinformationen nach Anspruch 1, das ferner den Schritt (D) der Klassifizierung der berechneten G-PSAD-Werte in zwei oder mehr Gruppen entsprechend der Größe der Werte umfasst.

3. Verfahren zur Gewinnung von Zusatzinformationen nach Anspruch 2, wobei der Schritt (D) ein Schritt des Klassifizierens der berechneten G-PSAD-Werte in drei Gruppen entsprechend der Größenordnung der Werte ist.

4. Verfahren zur Gewinnung von Zusatzinformationen nach einem der Ansprüche 1 bis 3, das ferner den Schritt (α) der Gewinnung eines Konzentrationswertes eines gesamten prostataspezifischen Antigens (Gesamt-PSA) umfasst, das in der aus dem Organismus stammenden Probe enthalten ist.

5. Verfahren zur Erfassung von Zusatzinformationen nach einem der Ansprüche 1 bis 4, wobei der Konzentrationswert des gesamten prostataspezifischen Antigens (Gesamt-PSA) in der aus dem lebenden Körper stammenden Probe größer als 0 und gleich oder kleiner als 100 ng/ml ist.

6. Verfahren zur Erfassung von Zusatzinformationen nach einem der Ansprüche 1 bis 4, wobei der Konzentrationswert des gesamten prostataspezifischen Antigens (Gesamt-PSA) in der vom Organismus stammenden Probe 2 bis 20 ng/ml beträgt.

7. Verfahren zur Gewinnung von Zusatzinformationen nach Anspruch 1, wobei das Molekül mit der Affinität für den β-N-Acetylgalactosamin-Rest Wisteria floribunda lectin (WFA), Sojabohnen-Agglutinin (SBA), Vicia Villosa lectin (VVL), Trichosanthes japonica agglutinin-II (TJA-II) oder ein Anti-β-N-Acetylgalactosamin-Antikörper ist.

8. Verfahren zur Erfassung von Zusatzinformationen nach Anspruch 1 oder 7, wobei Schritt (A) ein Schritt zur Messung des Konzentrationswerts des GalNAc-PSA durch Oberflächenplasmonen-verstärkte Fluoreszenzspektroskopie entspricht.

9. Ein Zusatzinformationserfassungssystem mit: einer Eingabevorrichtung; und einer Informationsverarbeitungsvorrichtung, wobei die Eingabevorrichtung eine Vorrichtung ist, in welche ein Konzentrationswert eines GalNAc-PSA in einer aus einem Organismus gewonnenen Probe und ein Volumenwert der Prostata des Organismus eingegeben wird, und
die Informationsverarbeitungsvorrichtung eine Vorrichtung ist, die Wahrscheinlichkeitsdaten für das Vorhandensein von Prostatakrebs auf der Grundlage von Informationen analysiert, die ein Ergebnis einschließen, das erhalten wird, indem der Konzentrationswert des GalNAc-PSA durch den Volumenwert der Prostata geteilt wird, um einen Wert (G-PSA-Dichte (G-PSAD)) der Konzentration des GalNAc-PSA pro Prostata-Volumeneinheit zu berechnen, wobei das Erfassungssystem das Verfahren zum Erfassen von Zusatzinformationen gemäß einem der Ansprüche 1 bis 8 durchführt.

10. Ein Programm, das einen Computer zur Durchführung des Erfassungsverfahrens nach einem der Ansprüche 1 bis 8 veranlasst.

## Revendications

1. Méthode pour acquérir des informations auxiliaires pour aider au diagnostic ou au traitement du cancer de la prostate, comprenant les étapes suivantes (A), (B) et (C),
étape (A) : étape d'acquisition d'une valeur de concentration d'un antigène spécifique de la prostate (GaINAc-PSA) ayant un résidu β-N-acétylgalactosamine à un terminal non réducteur d'une chaîne de sucre contenue dans un échantillon dérivé d'un corps vivant,
étape (B) : une étape d'acquisition d'une valeur de volume de la prostate du corps vivant, et
étape (C) : une étape de division de la valeur de la concentration du GalNAc-PSA par la valeur du volume de la prostate du corps vivant pour calculer une valeur (densité de G-PSA (G-PSAD)) de la concentration du GalNAc-PSA par volume de prostate, dans laquelle
l'étape (A) est une étape d'acquisition de la valeur de concentration du GalNAc-PSA par une interaction entre une molécule ayant une affinité pour le résidu β-N-acétylgalactosamine et le GalNAc-PSA.

2. La méthode pour acquérir des informations auxiliaires selon la revendication 1, comprenant en outre étape (D) de classification des valeurs G-PSAD calculées en deux groupes ou plus selon des ampleurs des valeurs.

3. La méthode pour acquérir des informations auxiliaires selon la revendication 2, dans laquelle
l'étape (D) est une étape de classification des valeurs G-PSAD calculées en trois groupes selon des ampleurs des valeurs.

4. La méthode pour acquérir des informations auxiliaires selon l'une quelconque des revendications 1 à 3, comprenant en outre étape (α) d'acquisition d'une valeur de concentration d'un antigène spécifique de la prostate total (PSA total) contenu dans l'échantillon dérivé du corps vivant.

5. La méthode pour acquérir des informations auxiliaires selon l'une quelconque des revendications 1 à 4, dans laquelle
la valeur de concentration de l'antigène spécifique de la prostate total (PSA total) dans l'échantillon dérivé du corps vivant est supérieure à 0 et égale ou inférieure à 100 ng/ml.

6. La méthode pour acquérir des informations auxiliaires selon l'une quelconque des revendications 1 à 4, dans laquelle
la valeur de concentration de l'antigène spécifique de la prostate total (PSA total) dans l'échantillon dérivé du corps vivant est de 2 à 20 ng/ml.

7. La méthode pour acquérir des informations auxiliaires selon la revendication 1, dans laquelle
la molécule ayant une affinité pour le résidu β-N-acétylgalactosamine est la lectine Wisteria floribunda (WFA), l'agglutinine de soja (SBA), la lectine Vicia Villosa (VVL), l'agglutinine-II Trichosanthes japonica (TJA-II), ou un anticorps anti-β-N-acétylgalactosam ine.

8. La méthode pour acquérir des informations auxiliaires selon la revendication 1 ou 7, dans lequel
l'étape (A) est une étape de mesure de la valeur de concentration du GalNAc-PSA par spectroscopie de fluorescence améliorée par champ plasmonique de surface.

9. Système d'acquisition d'informations auxiliaires comprenant : un dispositif d'entrée ; et un dispositif de traitement des informations,
dans lequel le dispositif d'entrée est un dispositif qui entre une valeur de concentration d'un GaINAc-PSA dans un échantillon dérivé d'un corps vivant et une valeur de volume de la prostate du corps vivant, et
le dispositif de traitement de l'information est un dispositif qui analyse des données de probabilité d'avoir un cancer de la prostate sur la base d'informations comprenant un résultat obtenu en divisant la valeur de concentration du GaINAc-PSA par la valeur du volume de la prostate pour calculer une valeur (densité G-PSA (G-PSAD)) de la concentration du GaINAc-PSA par unité de volume de la prostate, le système d'acquisition exécutant la méthode pour acquérir des informations auxiliaires selon l'une quelconque des revendications 1 à 8.

10. Un programme qui cause un ordinateur d'exécuter la méthode d'acquisition selon l'une quelconque des revendications 1 à 8.
